# FASCICULE DE BREVET EUROPEEN

(11) **EP 1 399 113 B1**
(45) Date de publication et mention de la délivrance du brevet: **21.03.2007**
(21) Numéro de dépôt: 02762497.2
(22) Date de dépôt: 26.06.2002
(51) Int. Cl.: A61Q 7/00, A61K 8/49, A61K 8/44, A61Q 19/08

(54) **COMPOSITION COSMETIQUE OU DERMATOLOGIQUE COMPRENANT DES DERIVES DES N-ACYLAMINOAMIDES**
KOSMETISCHE UND DERMATOLOGISCHE ZUSAMMENSETZUNG ENTHALTEND N-ACYLAMINOAMID-DERIVATE
COSMETIC OR DERMATOLOGICAL COMPOSITION COMPRISING N-ACYLAMINOAMIDE DERIVATIVES

(30) Priorité: 26.06.2001 FR 0108435
(43) Date de publication de la demande: 24.03.2004
(73) Titulaire: L'ORÉAL, 75008 Paris (FR)
(72) Inventeur: MAHE, Yann, F-91390 Morsang sur Orge (FR)
(74) Mandataire: Catherine, Alain
(86) Numéro de dépôt international: PCT/FR2002/002221
(87) Numéro de publication internationale: WO 2003/000209

(56) Documents cités:
- WO-A-00/12467
- FR-A- 2 606 635
- FR-A- 2 810 033
- US-A- 5 234 909
- PATENT ABSTRACTS OF JAPAN vol. 007, no. 185 (C-181), 13 août 1983 (1983-08-13) & JP 58 088307 A (TAKEO KINJI), 26 mai 1983 (1983-05-26)
- NAKAMURA M ET AL: "A two-step, one-pot synthesis of diverse N-pyruvoyl amino acid derivatives using the Ugi reaction" BIOORGANIC & MEDICINAL CHEMISTRY LETTERS, OXFORD, GB, vol. 10, no. 24, 18 décembre 2000 (2000-12-18), pages 2807-2810, XP004225356 ISSN: 0960-894X

## Description

L'invention est relative à des compositions cosmétiques ou dermatologiques destinées au traitement du cuir chevelu et des cheveux en particulier pour diminuer leur chute.

L'homme du métier sait depuis longtemps que la chute naturelle des cheveux, chez l'homme, reflète globalement l'équilibre des follicules pileux entre les phases alternatives de pousse (phase anagène) et les phases de chute (phase télogène). Le rapport moyen du nombre de follicules en phase anagène à celui en phase télogène est de l'ordre de 9 (90/10). Le pourcentage de follicules en phase de repos (phase catagène) y apparaît comme étant très faible.

La chute ou perte naturelle des cheveux peut être estimée, en moyenne, à quelques cent cheveux par jour pour un état physiologique normal. Pour un état alopécique , elle peut atteindre plusieurs centaines par jour conduisant à un dégarnissement modéré à important du cuir chevelu.

D'autre part, il existe à la surface du cuir chevelu une flore microbiologique naturellement constituée de bactéries et de levures. Lorsque qu'un déséquilibre intervient dans la composition naturelle de cette flore, la chute des cheveux peut être augmentée.

Il est connu, par ailleurs, que certains facteurs tels qu'un déséquilibre hormonal, un stress physiologique, les carences alimentaires, peuvent accélérer le phénomène.

Au cours de l'alopécie des zones micro-inflammatoires apparaissent dans le follicule pileux. Au cours de ce processus, de nombreux médiateurs de l'inflammation sont libérés par les différents sous-types cellulaires comme les kératinocytes des gaines externes et internes et/ou de la matrice, voire même dans certains cas par les fibroblastes de la papille.

Parmi ces médiateurs sont libérés des facteurs chimio-attractants sorte de " recruteurs biologiques" qui possèdent la capacité d'attirer certaines cellules inflammatoires du compartiment sanguin vers le tissu cutané. Ces cellules sont responsables ensuite de l'entretien d'une irritation locale.

On sait que lors d'un stress cutané superficiel, qui peut notamment être d'origine chimique, physique ou bactérienne, les kératinocytes folliculaires libèrent des médiateurs biologiques qui possèdent la capacité d'attirer certaines cellules infiltrantes de la peau, elles-mêmes responsables de l'entretien d'une irritation locale transitoire.

Parmi les médiateurs biologiques pouvant être produits par les kératinocytes ainsi stressés, on citera les chimiokines qui sont des cytokines chimioattractives responsables du recrutement de leucocytes sur les sites inflammatoires, dont l'interleukine 8 (IL-8) qui est plus particulièrement responsable du recrutement des neutrophiles.

Ces cellules infiltrant les zones irritées ou agressées libèrent alors des enzymes parmi lesquelles on peut citer l'élastase leucocytaire.
Sous l'action de cette enzyme notamment, les fibres élastiques de soutien extracellulaire du tissu conjonctif peuvent être dégradées, et entraîner ainsi une diminution de l'élasticité de la peau et de l'environnement péribulbaire.

Il est même par ailleurs connu qu'en synergie avec la cathepsine G, l'élastase leucocytaire peut dissocier l'intégrité de l'épiderme en élargissant les espaces intercellulaires interkératinocytaires.

Plus particulièrement pour le cuir chevelu, lorsque la dégradation est très importante, les fibres élastiques fines superficielles, perpendiculaires à la surface de la peau disparaissent et les fibres épaisses plus profondes, parallèles à la surface de la peau se fragmentent. On parle d'élastose actinique.

Lors d'un stress cutané (chimique, physique, bactérien ou neurogène) les kératinocytes libèrent des médiateurs biologiques (appelés facteurs chimioattractants) qui possèdent la capacité d'attirer certaines cellules inflammatoires du compartiment sanguin vers le tissu cutané. Ces cellules sont responsables de la genèse puis de l'entretien d'une irritation locale.

Parmi les facteurs chimio-attractants pouvant être produits par les kératinocytes stressés, l'interleukine 8 (IL-8) est plus spécifiquement responsables du recrutement des polynucléaires neutrophiles. Ces cellules infiltrant les zones irritées ou agressées libèrent alors des enzymes parmi lesquelles l' élastase leucocytaire et d'autres protéases (metalloprotéinases, serines protéases etc...)

Sous l'action de cette enzyme, les fibres élastiques de soutien extracellulaire du tissu conjonctif sont dégradées. En synergie avec la cathepsine G, l'élastase leucocytaire peut même dissocier l'intégrité de l'épiderme en élargissant les espaces intercellulaires inter-kératinocytaires (Ludolph-Hauser et al *Exp. Dermatol.* 1999 8(1) 46-52). L'élastase leucocytaire a récemment été incriminée dans l'entretien des escarres et la survenue des ulcères veineux des jambes, via son activité de dégradation de la fibronectine (Herrick S et al *Lab. Invest.* 1997(3) 281-288. La somme des micro-stress localisés de dégradation (suite par exemple à une exposition prolongée au soleil) peut aboutir au long terme à une perte accélérée de l'élasticité naturelle de la peau. Le réseau des fibres élastiques du tissu conjonctif sous jacent et des espaces extracellulaires est alors progressivement déstructuré. Cette dégradation accélérée peut se cumuler avec le processus de vieillissement normal de la peau qui se caractérise par une plus grande sensibilité des fibres élastiques à l'action de l'élastase (Stadler R & Orfanos CE *Arch. Dermatol. Res.* 1978 262 (1) 97-111

Il est connu dans l'état de la technique d'apporter, dans le tissu cutané, des molécules capables de ralentir l'activité de dégradation des fibres élastiques des espaces intercellulaires.

En particulier, il est connu dans l'état de la technique d'apporter au niveau de la zone alopécique, un agent limitant la chute du cheveu . Parmi ces ingrédients cosmétiques et/ou dermatologiques, citons les vitamines, la Biotine, l' Aminexil, le Minoxidil, les agents énergétiques sucrés, le ginseng, **le tripeptide KPV et ses sels,** Il existe aussi des solutions intervenant par voie systémique sur le métabolisme des androgènes (Finasteride®).

La solution technique selon l'invention consiste à associer aux actifs anti-chute / repousse du cheveu quel que soit leur mode d'administration et leur mode d'action, un inhibiteur de l'activité élastasique capable de ralentir aussi l'activité de dégradation des fibres élastiques des espaces intercellulaires du cuir chevelu.

En conséquence, l'invention a pour objet une composition cosmétique ou dermatologique caractérisée en ce qu'elle comprend une association entre un composé inhibiteur de l'élastase de la famille des N-Acylaminoamides tel que défini dans la revendication 1 et au moins un composé diminuant la chute des cheveux et/ou favorisant la repousse des cheveux.

Un autre objet de l'invention consiste en un procédé de traitement cosmétique des cheveux et/ou du cuir chevelu, caractérisé par le fait que l'on applique sur les cheveux et/ou le cuir chevelu une composition telle que définie ci-dessus.

Il a en effet été constaté que les composés de formule (I) tels que définis dans la revendication 1 présentaient une activité inhibitrice de l'activité des élastases, et qu'ils pouvaient donc être employés pour limiter et/ou combattre la dégradation des fibres élastiques.

Il s'en suit qu'ils peuvent être employés dans ou pour la préparation d'une composition, les composés ou la composition étant destinés à traiter, de manière préventive et/ou curative, la dégradation des fibres élastiques des espaces intercellulaires, particulièrement du cuir chevelu.

L'association nouvelle des composés N-Acylaminoamides tels que définis dans la revendication 1 avec au moins un composé diminuant la chute des cheveux et/ou favorisant la repousse des cheveux permet de renforcer significativement l'effet antivieillissement du tissu matriciel, par l'apport d'un effet anti-chute de cheveux ou d'un effet de repousse des cheveux.

Selon l'invention, l'élément régulateur de l'activité élastasique, le composé N-acyloaminoamide, et préférentiellement un composé tel que défini dans la revendication 1, est combiné à un ou plusieurs actifs capables diminuer la chute des cheveux ou de favoriser la repousse des cheveux.

La composition obtenue est destinée à traiter les désordres alopéciques et/ou de repousse des cheveux .

Préférentiellement, cette nouvelle association est utilisée dans des préparations cosmétiques de soin et/ou d'hygiène des zones exposées au soleil (scalp, cuir chevelu), dans les préparation cosmétiques de soin et/ou d'hygiène des zones capillaires et, de façon générale, dans toutes les préparations cosmétiques dites " anti-vieillissement » qui ont pour objectif de ralentir la chute des cheveux ou de favoriser leur repousse.

Sans vouloir être lié par une quelconque théorie, le demandeur considère que le fait d'apporter, au niveau des kératinocytes des couches superficielles de la peau et/ou de la région péribulbaire, des composés susceptibles de ralentir l'activité de dégradation des fibres élastiques ., peut permettre de diminuer ce phénomène de vieillissement accéléré de la peau, dû à des stress cutanés superficiels et que l'association de ces composés avec au moins un composé diminuant la chute des cheveux et/ou favorisant la repousse des cheveux renforce considérablement leurs effets.

### Composés N-acylamino-amides préférés

Les composés employés dans la présente invention répondent à la formule (I) suivante : 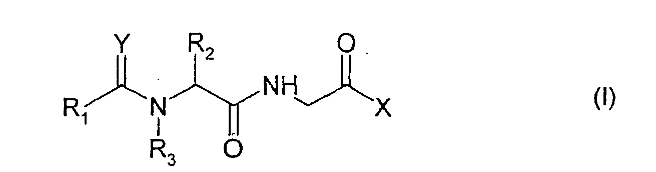 dans laquelle :
- le radical Y représente l'oxygène, et/ou
- le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué, et/ou
- les substituants de R1 sont choisis parmi -OH, -OR et/ou -P(O)-(OR)₂ avec R représentant un radical hydrocarboné linéaire, ramifié ou cyclique, saturé ou insaturé, ayant 1 à 6 atomes de carbone, éventuellement halogéné, voire perhalogéné; et/ou
- le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué; et/ou
- les substituants de R2 sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2; et/ou
- le radical A de R3 est un radical divalent hydrocarboné, linéaire ou ramifié, saturé ou insaturé, ayant 1 à 12 atomes de carbone, éventuellement substitué; et/ou
- le radical B de R3 est un radical hydrocarboné, linéaire ou ramifié, saturé ou insaturé, ayant 1 à 12 atomes de carbone, éventuellement substitué; et/ou
- les substituants de A et/ou de B sont choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné; et/ou
- le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué; et/ou
- les substituants de R4 de X sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné ;
ses sels d'acide minéral ou organique, ses isomères optiques, sous forme isolée ou en mélange racémique ;

Par radical hydrocarboné, linéaire, cyclique ou ramifié, on entend notamment les radicaux de type alkyle, aryle, aralkyle, alkylaryle, alcényle, alcynyle.

Le groupement C₆H₅ présent dans le radical R3 doit être compris comme un groupement cyclique aromatique.

Préférentiellement, le radical R1 représente un radical méthyle, éthyle, propyle ou isopropyle, éventuellement substitué par un groupement -OH ou - P(O)-(OR)₂ avec R représentant méthyle, éthyle, propyle ou isopropyle.

Préférentiellement, le radical R2 représente un radical méthyle, éthyle, propyle, isopropyle, n-butyle, ter-butyle ou isobutyle.

Préférentiellement, le radical R3 représente un groupement choisi parmi l'une des formules suivantes : 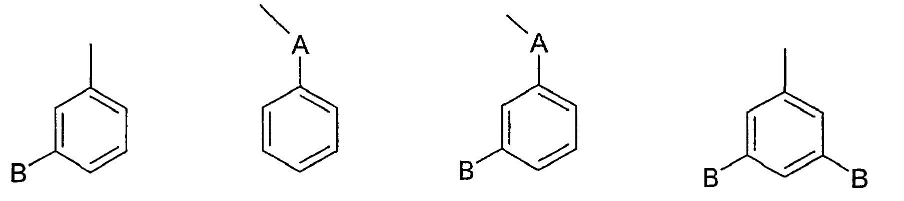 dans lesquelles A et B ont les significations ci-dessus.

Notamment, le radical divalent A peut être un méthylène, un éthylène, un propylène.

Le radical B est de préférence un radical méthyle, éthyle, propyle ou isopropyle, substitué par un ou plusieurs halogènes, notamment chlore, brome, iode ou fluor, et préférentiellement totalement halogéné (perhalogéné), tel que perfluoré. On peut en particulier citer le radical perfluorométhyle (-CF3) comme tout particulièrement préféré.

Les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical X représente un radical choisi parmi -OH, - OCH₃, -OC₂H₅, -O-C₃H₇ ou -OC₄H₉.

Parmi les composés particulièrement préférés, on peut citer :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.

Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, notamment entre 0,001 et 10% en poids, et préférentiellement entre 0,5 et 1 % en poids.

L'association des composés de formule (I) avec un composé diminuant la chute des cheveux et/ou favorisant la repousse des cheveux peut notamment être employée dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

### Composés anti-chute de cheveux préférés selon l'invention

Les composés diminuant la chute des cheveux ou favorisant la repousse des cheveux selon l'invention sont préférentiellement les agents améliorant la viabilité du follicule pileux.

Les composés préférés sont les vitamines, telles que la Biotine, l' Aminexil, le minoxidil, le tripeptide KPV et ses sels, les agents énergétiques sucrés et le ginseng.

D'autres composés préférés consistent en des composés agissant par voie systémique sur le métabolisme des androgènes, tels que le Finasteride®.

Les agents anti-chute de cheveux sont présents de préférence à une concentration comprise entre 0,01% et 10 % en poids total de la composition, de préférence entre 0,1 % et 5%

L'association d'au moins un composé N-acylamino-amide tel que défini dans la revendication 1 et d'au moins un composé diminuant la chute des cheveux et/ou favorisant la repousse des cheveux peut notamment être employée, seule ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.

Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.

D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.

Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Les compositions conformes à l'invention peuvent se présenter sous des formes diverses habituellement utilisées en cosmétique ou en dermatologie pour le traitement du cuir chevelu.

Elles peuvent se présenter plus particulièrement sous forme de lotions, de shampooings, de mousses, de crèmes, de gels, de sticks, de sprays, de baumes, de poudres, de savons solides ou liquides.

Le milieu physiologiquement acceptable est généralement constitué par de l'eau ou par un mélange d'eau et d'au moins un solvant organique acceptable d'un point de vue physiologique en vue d'une application topique. Parmi ces solvants, on peut mentionner l'acétone, les alcools inférieurs en C₁-C₄ tels que l'éthanol, l'alcool isopropylique, les alkylène-glycol tel que l'éthylèneglycol, le propylène-glycol, les éthers monométhylique, monoéthylique ou monobutylique de l'éthylèneglycol, les monoéthyléthers du propylèneglycol et du dipropylèneglycol, les esters d'alkyle en C₁-C₄ d'acide à chaîne courte et les éthers de polytétrahydrofuranne. Lorsqu'ils sont présents, ces solvants représentent de préférence de 1 à 80 % en poids du poids total de la composition.

Le milieu peut être épaissi à l'aide d'agents épaississants habituellement utilisés en cosmétique ou en pharmacie.

Parmi ces agents épaississants, on peut en particulier citer la cellulose et ses dérivés comme les éthers de cellulose, les hétérobiopolysaccharides tels que la gomme de xanthane, les scléroglucanes, les acides polyacryliques réticulés ou non.

Les agents épaississants sont présents de préférence dans des proportions variant entre 0,1 et 5 % en poids environ par rapport au poids total de la composition.

Suivant les diverses formes de présentation désirées des compositions, l'homme de métier saura choisir les composés et adjuvants nécessaires et habituellement utilisés pour la réalisation de ces compositions.

Parmi ces adjuvants, on peut notamment citer les agents conservateurs, les agents stabilisants, les agents régulateurs de pH, les agents modificateurs de pression osmotique, les agents émulsifiants, les filtres solaires, les agents antioxydants, les parfums, les colorants, les agents tensioactifs, anioniques, cationiques, non-ioniques, amphotères, zwittérioniques ou leurs mélanges, les polymères, etc...

Les compositions peuvent également contenir en plus de l'association particulière faisant l'objet de l'invention, des composés déjà connus pour freiner la chute des cheveux.

De manière préférée, une composition selon l'invention peut être utilisée dans des préparations cosmétiques de soin antichute / repousse des zones alopéciques particulièrement lorsqu'elles sont exposées au soleil. En effet, la diminution relative de la densité pilaire qui apparaît au cours de l'alopécie concourt à une protection UV moindre - de fait, la composante inflammatoire de cette alopécie peut être maximalisée par une plus grande exposition au rayonnement UV.

L'invention a également pour objet un procédé de traitement cosmétique des cheveux et/ou du cuir chevelu consistant à leur appliquer une composition telle que définie ci-dessus, en vue de diminuer leur chute.

Le mode d'application préféré consiste à appliquer 0.1 à 20 g de la composition, sur l'ensemble ou sur certaines parties du cuir chevelu, à une fréquence de une à deux applications par jour, pendant 1 à 7 jours par semaine et ceci pendant une durée de 1 à 9 mois renouvelable selon l'évolution de la progression de l'alopécie.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection du cuir chevelu, tel qu'une composition capillaire, et notamment une crème ou un gel protecteur solaire; une composition de soin du cuir chevelu, notamment anti-chute ou repousse des cheveux; un shampooing antiparasitaire ou apaisant L'invention a également pour objet un procédé de traitement cosmétique des cheveux et/ou du cuir chevelu consistant à leur appliquer une composition telle que définie ci-dessus, en vue de diminuer leur chute.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de shampoings, de laits de démaquillage ou de compositions de protection solaires ou après solaires sur la peau ou sur les cheveux secs; application d'une lotion pour cuir chevelu sur cheveux mouillés; .

Le mode d'application préféré consiste à appliquer 1 à 20 g de la composition, sur l'ensemble ou sur certaines parties du cuir chevelu, à une fréquence de une à deux applications par jour, pendant 1 à 7 jours par semaine et ceci pendant une durée de 1 à 9 mois renouvelable selon le degré d'exposition à l'agent de stress et selon le degré d'alopécie.

Les exemples suivants sont destinés à illustrer l'invention sans pour autant en limiter la portée.

### Exemple 1

Préparation du {2-[acétyl-(3-trifluorométhyl-phényl)-amino]-3-méthyl-butyrylamino} acétate d'éthyle de formule :

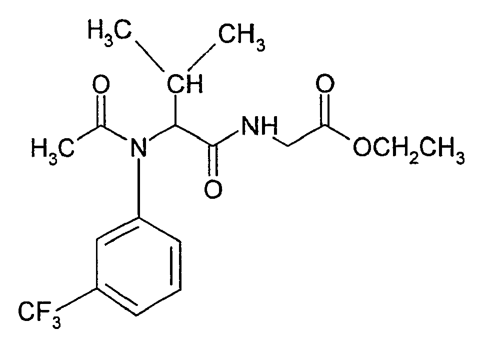

On mélange 0,63 ml d'isobutyraldéhyde et 1 ml de trifluorométhylamine (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.

On concentre le milieu réactionnel au rotovapor et on purifie le résidu sur colonne de silice (éluant : heptane : 3 / acétate d'éthyle : 7; Rf = 0,5).

On obtient 2,45 g de composé sous forme de solide cireux, d'où un rendement de 91%.
RMN ¹H (200 MHz; CDCl3) δ ppm : 0,9 (6H;q), 1,3 (3H;t), 1,8 (3H;s), 2,3 (1H;m), 4,0 (2H,q), 4,2 (2H;q), 4,4 (2H;d), 7,3 (1 H;t), 7,5 (4H;m)

### Exemple 2

### Préparation de l'acide {2-[acétyl-(3-trifluorométhyl-phényl)-amino]-3-méthyl-butyrylamino} acétique de formule :

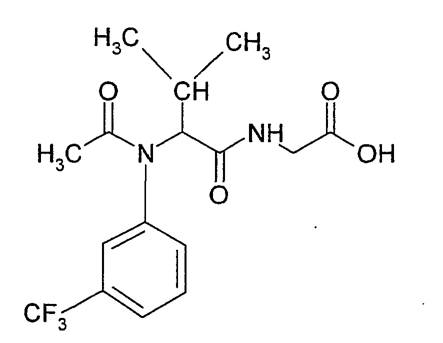

On solubilise 2 g de composé préparé à l'exemple 1 dans 30 ml d'acétone. On ajoute 30 ml de soude 2N et on laisse réagir pendant 6 heures à 20°C. On concentre le milieu réactionnel au rotovapor. On acidifie la phase aqueuse résiduelle à pH 2 par ajout d'HCl concentré puis on extrait par CH2Cl2.

La phase organique est concentrée à sec après séchage sur sulfate de sodium.

On obtient un résidu qui est solubilisé par un mélange eau basique à 10% d'éthanol, puis de nouveau acidifié par HCl concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.
RMN ¹H (200 MHz; DMSO) δ ppm : 0,9 (6H;q), 3,7 (2H;m), 1,8 (4H;m), 4,8 (2H;d), 7,6 (4H,m),8,4 (1 H;t), 12,5 (1 H;s)

### Exemple 3

On a déterminé in vitro l'activité anti-élastasique de composés selon l'invention, vis-à-vis de l'élastase leucocytaire humaine (ELH).

Le test est effectué de la manière suivante :

On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1% du composé à tester.

On détermine ensuite par spectrophotométrie le % d'inhibition de l'activité élastase témoin.

Les composés testés sont les suivants :
Composé A : acide {2-[acétyl-(3-trifluorométhyl-phényl)-amino]-3-méthyl-butyryl-amino} acétique
Composé B : (2-{benzyl-[(diethoxy-phosphoryl)-acétyl]-amino}-3-méthyl-butyryl-amino) acétate d'éthyle
Composé C : acide [2-(acétyl-benzyl-amino)-3-méthyl-butyrylamino] acétique
Composé D : [2-(acétyl-benzyl-amino)-3-méthyl-butyrylamino] acétate d'éthyle

On obtient les résultats suivants :

| Composé (concentration : 0,1%) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01% | 53% |
| 0,05% | 50% |
| 0,1 % | 68% |
| 0,2% | 68% |

Le composé A provoque donc une forte inhibition de l'activité élastase, même en une quantité faible.

### Exemple 4

On a évalué l'activité ex vivo du composé de l'exemple 2 sur des peaux humaines en survie traitées par de l'élastase leucocytaire humaine (ELH).

Le test est effectué de la manière suivante :

Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Exemple 5

On a évalué l'activité ex vivo du composé de l'exemple 2 sur des peaux humaines en survie traitées par de l'élastase leucocytaire humaine (ELH).

Le test est effectué de la manière suivante :

Des fragments de peau humaine normale provenant de trois donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau éventuellement 0,5 µg d'ELH par ml de milieu de culture.

On ajoute également, tous les deux jours, 5 µl du composé à tester, préalablement mis en solution à 0,2% en poids dans l'éthanol.

Les peaux sont maintenues en survie pendant 10 jours à 37°C.

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1% |

On constate donc que le composé selon l'invention génère une protection significative des peaux vis-à-vis de la destruction des fibres élastiques induite par l'élastase.

### Exemple 6

On a évalué l'activité du composé de l'exemple 2 sur des peaux humaines en survie irradiées par des UVA (8 J/cm²).

Le test est effectué de la manière suivante :

Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).

Le milieu de culture est renouvelé tous les trois jours.

On ajoute sur les fragments de peau, tous les deux jours, 5 µl du composé à tester, préalablement mis en solution à 0,2% dans l'éthanol.

Les peaux sont maintenues en survie pendant 7 jours à 37°C.

Les peaux sont irradiées une seule fois à 8 J/cm² (lampe Vilbert-Lourmat RMX-3W).

Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | Analyse morphométrique des fibres élastiques (derme superficiel) | Analyse morphométrique du collagène (derme superficiel) |
|---|---|---|
| Peau non traitée | 6,75% | 87% |
| Peau traitée par des UVA (8 J/cm²) | 3,9% | 81% |
| Peau traitée par des UVA (8 J/cm²) + composé | 6,8% | 92% |

On constate que le composé selon l'invention a bien une activité vis-à-vis de la dégradation des fibres élastiques dans le derme superficiel de peaux irradiées par des UVA.

### Exemple 7 : lotion capillaire

On prépare la lotion suivante de façon classique (% en poids) :
- composé de l'Exemple 1 1 %
- propylène glycol 23 %
- éthanol 55 %
- Hydrocortisone 0.1 %
- Minoxidil 1,5 %

On peut appliquer cette lotion sur le scalp des individus alopéciques, pour prévenir les effets des UV, avant et/ou après exposition au soleil.

### Exemple 8 : lotion antichute (hors invention)

On prépare la lotion suivante de façon classique (% en poids) :
- composé de l'Exemple 2 1 %
- propylène glycol 23 %
- éthanol 55 %
- Aminexil 1,5%
- eau qsp 100 %

On peut appliquer cette lotion antichute sur le scalp des individus alopéciques.

## Revendications

1. Composition cosmétique ou dermatologique **caractérisée en ce qu'**elle comprend une association entre un composé inhibiteur de l'élastase de la famille des N-Acylaminoamides et au moins un composé diminuant la chute des cheveux et/ou favorisant la repousse des cheveux, le composé inhibiteur de la famille des N-Acylaminoamides étant un composé de formule (I) : 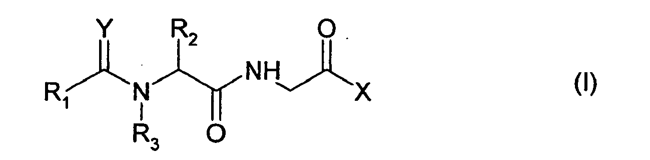 dans laquelle :
- le radical Y représente l'oxygène, et/ou
- le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué, et/ou
- les substituants de R1 sont choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué; et/ou
- les substituants de R2 sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R3 représente un radical de formule -C₆H_{(5-y')}-B_{y'} pour lequel y' = 1, 2 ou 3; ou un radical de formule -A-C₆H_{(5-y)}-B_{y} pour lequel y = 0, 1 ou 2; et/ou
- le radical A de R3 est un radical divalent hydrocarboné, linéaire ou ramifié, saturé ou insaturé, ayant 1 à 12 atomes de carbone, éventuellement substitué; et/ou
- le radical B de R3 est un radical hydrocarboné, linéaire ou ramifié, saturé ou insaturé, ayant 1 à 12 atomes de carbone, éventuellement substitué; et/ou
- les substituants de A et/ou de B sont choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné; et/ou
- le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué; et/ou
- les substituants de R4 de X sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné ;
ses sels d'acide minéral ou organique, ses isomères optiques, sous forme isolée ou en mélange racémique ;
étant entendu que la composition ne comprend pas l'association de l'acide {2-[acétyl-(3-trifluorométhyl-phényl)-amino]-3-méthyl-butyrylamino} acétique et de l'Aminexil.

2. Composition selon la revendication 1, dans laquelle le composé de formule (I) est tel que :
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 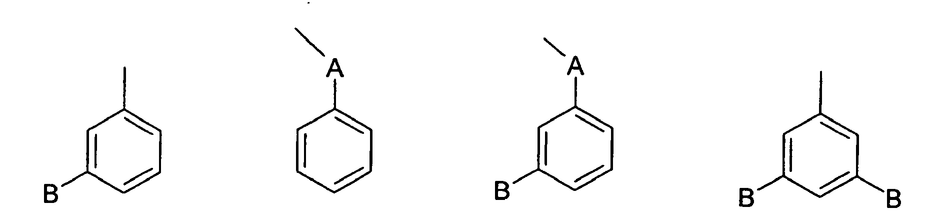 dans lesquelles le radical divalent A est un méthylène, un éthylène, un propylène et/ou le radical B est un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -O-C₃H₇ ou -OC₄H₉.

3. Composition selon l'une des revendications 1 ou 2, dans laquelle le composé de formule (I) est choisi parmi les composés suivants :
- l'acide {2-[acétyl-(3-trifluorométhyl-phényl)-amino]-3-méthyl-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl)-acétyl]-amino}-3-méthyl-butyrylamino) acétate d'éthyle.

4. Composition selon l'une des revendications précédentes, dans laquelle le composé inhibiteur de l'élastase de la famille des N-Acylaminoamides est présent en une quantité comprise entre 0,00001 et 20% en poids par rapport au poids total de la composition, notamment entre 0,001 et 10% en poids, et préférentiellement entre 0,5 et 1 % en poids.

5. Composition selon l'une des revendications précédentes, **caractérisée en ce que** le composé diminuant la chute des cheveux ou favorisant la repousse des cheveux est choisi parmi les agents améliorant la viabilité du follicule pileux.

6. Composition selon l'une des revendications 1 à 4, **caractérisée en ce que** le composé diminuant la chute des cheveux ou favorisant la repousse des cheveux est choisi parmi les vitamines, les agents énergétiques sucrés et le ginseng.

7. Composition selon l'une des revendications 1 à 4, **caractérisée en ce que** le composé diminuant la chute des cheveux ou favorisant la repousse des cheveux est choisi parmi la Biotine, l'aminexil, le minoxidil et le tripeptide KPV et ses sels.

8. Composition selon l'une des revendications 1 à 4, **caractérisée en ce que** le composé diminuant la chute des cheveux ou favorisant la repousse des cheveux est choisi parmi les composés agissant par voie systémique sur le métabolisme des androgènes.

9. Composition selon l'une des revendications 1 à 4, **caractérisée en ce que** le composé diminuant la chute des cheveux ou favorisant la repousse des cheveux est présent de préférence à une concentration comprise entre 0,01% et 10 % en poids total de la composition, de préférence entre 0, 1 % et 5%.

10. Procédé de traitement cosmétique des cheveux et/ou du cuir chevelu, **caractérisé par le fait que** l'on applique sur les cheveux et/ou le cuir chevelu une composition selon l'une des revendications 1 à 9.

## Claims

1. A cosmetic or dermatological composition **characterized in that** it comprises a combination between an elastase inhibiting compound of the N-acylaminoamides and at least one compound reducing the hair loss and/or enhancing the hair regrowth the inhibiting compound of the N-acylaminoamides is a compound of formula (I) : 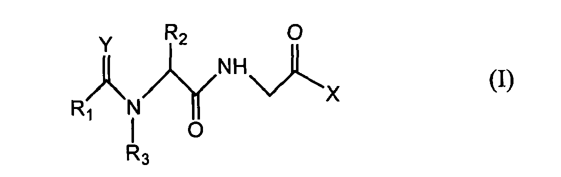 wherein :
- the Y radical is oxygen, and/or
- the R1 radical is hydrogen or a linear or branched, saturated or unsaturated hydrocarbon radical, having from 1 to 12, and more preferably, 1, 2, 3, 4, 5 or 6 carbon atoms, optionally substituted and/or
- the R1 substituents are choosen amongst -OH, -OR and/or - P(O)-(OR)₂ with R representing a linear, branched or cyclic, saturated or unsaturated hydrocarbon radical having from 1 to 6 carbon atoms, optionally halogenated, even perhalogenated and/or
- the R2 radical is a linear, branched or cyclic, saturated or unsaturated hydrocarbon radical having from 1 to 12, preferably 1, 2, 3, 4, 5 or 6 carbon atoms, optionally substituted and/or
- the substituents for R2 are choosen amongst -OH and -OR with R representing a linear, branched or cyclic, saturated or unsaturated hydrocarbon radical having 1 to 6 carbon atoms, optionally halogenated, even perhalogenated; and/or
- the R3 radical is a radical of formula -C₆H_{(5-y')}-B_{y'} wherein y' = 1, 2 or 3; or a radical of formula -A-C₆H_{(5-y)}-B_{y} wherein y = 0, 1 or 2 and/or
the A radical for R3 is a linear or branched, saturated or unsaturated divalent hydrocarbon radical having 1 to 12 carbon atoms, optionally substituted and/or
the B radical for R3 is a linear or branched, saturated or unsaturated hydrocarbon radical having 1 to 12 carbon atoms, optionally substituted; and/or
the substituents for A and/or B are choosen amongst -Hal (halogen, even perhalogen); -CN; -COOR; -NO₂; -SO₂-OR; with R representing a linear, branched or cyclic, saturated or unsaturated hydrocarbon radical having 1 to 6 carbon atoms, optionally halogenated, or even perhalogenated and/or
the X radical is a radical choosen amongst -OH or OR₄ with R₄ representing a linear, cyclic or branched, saturated or unsaturated hydrocarbon radical having 1 to 6 carbon atoms, optionally substituted and/or
the R4 substituents for X are choosen amongst -OH and -OR with R representing a linear, branched or cyclic, saturated or unsaturated hydrocarbon radical having 1 to 6 carbon atoms, optionally halogenated, even perhalogenated.
The inorganic or organic acid salts thereof, the optical isomers thereof isolated or in a racemic mixture while the composition does not include the combination of (2-[acetyl-(3-trifluoromethyl-phenyl)-amino]-3-methyl-butyrylamino) acetic acid and aminexil.

2. A composition according to claim 1, wherein the compound of formula (I) is such that:
- the R1 radical is a methyl, ethyl, propyl or isopropyl radical optionally substituted by a -OH or -P(O)-(OR)₂ group with R representing a methyl, ethyl, propyl or isopropyl radical; and/or
- the R2 radical is a methyl, ethyl, propyl, isopropyl, n-butyl, tert-butyl or isobutyl radical; and/or
- the R3 radical is a group choosen amongst one of the following formulae : 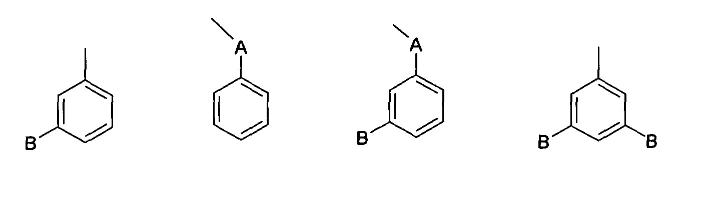
wherein the A divalent radical is a methylene, an ethylene, a propylene radical and/or the B radical is a methyl, ethyl, propyl or isopropyl radical, substituted by one or more halogens, more particularly, chlorine, bromine, iodine or fluorine, and preferably wholly halogenated (perhalogenated), such as perfluorated, more particularly the perfluoromethyl radical (-CF3),
- the X radical is a radical choosen amongt -OH, -OCH₃, -OC₂H₅, -O-C₃H₇ or -OC₄H₉.

3. A composition according to one of claims 1 or 2, wherein the compound of formula (I) is choosen amongst the following compounds :
- the {2-[acetyl-(3-trifluoromethyl-phenyl)-amino]-3-methyl-butyrylamino} acetic acid,
- the ethyl {2-[acetyl-(3-trifluoromethyl-phenyl)-amino]-3-methyl-butyrylamino} acetate,
- the [2-(acetyl-benzyl-amino)-3-methyl-butyrylamino] acetic acid,
- the ethyl [2-(acetyl-benzyl-amino)-3-methyl-butyrylamino] acetate,
- the ethyl (2-{benzyl-[(diethoxy-phosphoryl)-acetyl]-amino}-3-methyl-butyrylamino] acetate.

4. A composition according to one of the preceding claims, wherein the elastase inhibiting compound from the N-acylaminoamides in an amount ranging from 0.00001 to 20% by weight based on the total weight of the composition, more preferably from 0.001 to 10% by weight, and most preferably, from 0.5 to 1% by weight.

5. A composition according to one of the preceding claims, **characterized in that** the compound for reducing the hair loss or enhancing the hair regrowth is choosen amongst agents improving the viability of the hair follicle.

6. A composition according to one of claims 1 to 4, **characterized in that** the compound for reducing the hair loss or enhancing the hair regrowth is choosen amongst vitamins, sugar energetic agents and ginseng.

7. A composition according to one of claims 1 to 4, **characterized in that** the compound for reducing the hair loss or enhancing the hair regrowth is choosen amongst Biotin, Aminexil, minoxidil, KPV tripeptide and the salts thereof.

8. A composition according to one of claims 1 to 4, **characterized in that** the compound for reducing the hair loss or enhancing the hair regrowth is choosen amongst compounds systemically acting on the androgen metabolism.

9. A composition according to one of claims 1 to 4, **characterized in that** the compound for reducing the hair loss or enhancing the hair regrowth is preferably in an amount ranging from 0.01 to 10% based on the total weight of the composition, preferably from 0.1 to 5%.

10. A method for cosmetically treating the hair and/or the hair scalp, **characterized in that** a composition according to one of claims 1 to 9 is applied on the hair and/or on the hair scalp.

## Patentansprüche

1. Kosmetische oder dermatologische Zusammensetzung, **dadurch gekennzeichnet , dass** sie eine Assoziation zwischen einer Inhibitorverbindung der Elastase aus der Familie der N-Acylaminoamide und wenigstens einer Verbindung, die den Haarausfall verringert und/oder das (Nach)Wachsen der Haare begünstigt, umfasst, wobei die Inhibitorverbindung aus der Familie der N-Acylaminoamide eine Verbindung der Formel (I) ist: 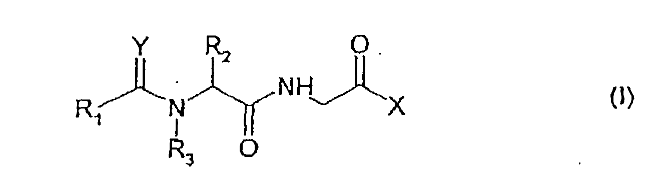 worin:
- der Rest Y Sauerstoff darstellt und/oder
- der Rest R1 Wasserstoff oder einen linearen oder verzweigten, gesättigten oder ungesättigten Kohlenwasserstoffrest darstellt, der 1 bis 12 und insbesondere 1, 2, 3, 4, 5 oder 6 Kohlenstoffatome hat, der gegebenenfalls substituiert ist, und/oder
- die Substituenten von R1 ausgewählt sind unter -OH, -OR und/oder -P(O)-(OR)₂, wobei R einen linearen, verzweigten oder cyclischen, gesättigten oder ungesättigten Kohlenwasserstoffrest darstellt, der 1 bis 6 Kohlenstoffatome hat, der gegebenenfalls halogeniert, sogar perhalogeniert ist; und/oder
- der Rest R2 einen linearen, verzweigten oder cyclischen, gesättigten oder ungesättigten Kohlenwasserstoffrest darstellt, der 1 bis 12, insbesondere 1, 2, 3, 4, 5 oder 6 Kohlenstoffatome hat, der gegebenenfalls substituiert ist; und/oder
- die Substituenten von R2 ausgewählt sind unter -OH und -OR, wobei R einen linearen, verzweigten oder cyclischen, gesättigten oder ungesättigten Kohlenwasserstoffrest darstellt, der 1 bis 6 Kohlenstoffatome hat, der gegebenenfalls halogeniert, sogar perhalogeniert ist; und/oder
- der Rest R3 einen Rest der Formel -C₆H_{(5-y')}-B_{y'}, für die y' = 1,2 oder 3, oder einen Rest der Formel -A-C₆H_{(5-y)}-By, für die y = 0,1 oder 2 ist, darstellt; und/oder
- der Rest A von R3 ein linearer oder verzweigter, gesättigter oder ungesättigter zweiwertiger Kohlenwasserstoffrest ist, der 1 bis 12 Kohlenstoffatome hat, der gegebenenfalls substituiert ist; und/oder
- der Rest B von R3 ein linearer oder verzweigter, gesättigter oder ungesättigter Kohlenwasserstoffrest ist, der 1 bis 12 Kohlenstoffatome hat, der gegebenenfalls substituiert ist; und/oder
- die Substituenten von A und/oder von B ausgewählt sind unter -Hal (Halogen, sogar Perhalogen); -CN; -COOR; -NO₂; -SO₂-OR; wobei R einen linearen, verzweigten oder cyclischen, gesättigten oder ungesättigten Kohlenwasserstoffrest darstellt, der 1 bis 6 Kohlenstoffatome hat, der gegebenenfalls halogeniert, sogar perhalogeniert ist; und/oder
- der Rest X einen Rest darstellt, ausgewählt unter -OH und -OR₄, wobei R₄ einen linearen, cyclischen oder verzweigten, gesättigten oder ungesättigten Kohlenwasserstoffrest darstellt, der 1 bis 6 Kohlenstoffatome hat, der gegebenenfalls substituiert ist; und/oder
- die Substituenten R4 von X ausgewählt sind unter -OH und -OR, wobei R einen linearen, verzweigten oder cyclischen, gesättigten oder ungesättigten Kohlenwasserstoffrest darstellt, der 1 bis 6 Kohlenstoffatome hat, der gegebenenfalls halogeniert, sogar perhalogeniert ist;
seine Salze mit Mineralsäure oder organischer Säure, ihren optischen Isomeren, in isolierter Form oder als razemisches Gemisch;
mit der Maßgabe, dass die Zusammensetzung nicht die Assoziation der {2-[Acetyl-(3-trifluormethylphenyl)-amino]-3-methyl-butyrylamino}essigsäure und Aminexil umfasst.

2. Zusammensetzung nach Anspruch 1, in der die Verbindung der Formel (I) die ist, in der:
- der Rest R1 einen Methyl-, Ethyl-, Propyl- oder Isopropylrest darstellt, der gegebenenfalls mit einer Gruppierung -OH oder -P(O)-(OR)₂ substituiert ist, wobei R Methyl, Ethyl, Propyl oder Isopropyl darstellt; und/oder
- der Rest R2 einen Methyl-, Ethyl-, Propyl-, Isopropyl-, n-Butyl-, tert.-Butyl oder Isobutyl-Rest darstellt; und/oder
- der Rest R3 eine Gruppierung darstellt, die ausgewählt ist unter den folgenden Formeln: 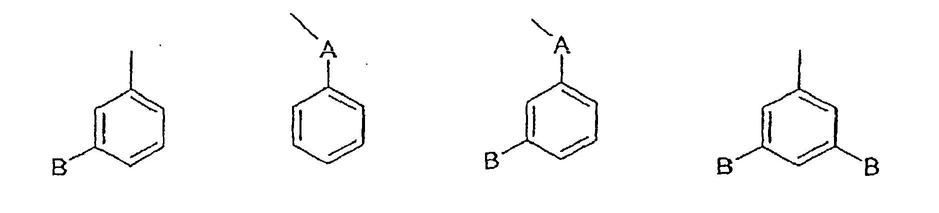 in denen der zweiwertige Rest A ein Methylen, ein Ethylen, ein Propylen ist und/oder der Rest B ein Methyl-, Ethyl-, Propyl- oder Isopropylrest ist, der durch ein oder mehrere Halogene, insbesondere Chlor, Brom, Iod oder Fluor substituiert ist und vorzugsweise vollständig halogeniert (perhalogeniert) ist, wie perfluoriert ist, insbesondere der Perfluormethylrest (-CF3) ist;
- der Rest X einen Rest darstellt, der aus -OH, -OCH₃, -OC₂H₅, -O-C₃H₇ und -OC₄H₉ ausgewählt ist.

3. Zusammensetzung nach einem der Ansprüche 1 und 2, wobei die Verbindung der Formel (I) unter den folgenden Verbindungen ausgewählt ist:
{2-[Acetyl-(3-trifluormethyl-phenyl)-amino]-3-methyl-butyryl-amino}essigsäure,
{2-[Acetyl-(3-trifluormethyl-phenyl)-amino]-3-methyl-butyryl-amino}essigsäureethylester,
[2-(Acetyl-benzyl-amino)-3-methyl-butyrylamino]essigsäure,
[2-(Acetyl-benzyl-amino)-3-methyl-butyrylamino]essigsäureethylester
(2-{Benzyl-[(diethoxy-phosphoryl)-acetyl]-amino}-3-methyl-butyrylamino)essigsäureethylester.

4. Zusammensetzung nach einem der vorangehenden Ansprüche, in der die Inhibitorverbindung der Elastase aus der Familie der N-Acylaminoamide in einer Menge zwischen 0,00001 und 20 Gew.-%, bezogen auf das Gesamtgewicht der Zusammensetzung, insbesondere zwischen 0,001 und 10 Gewichts-% und vorzugsweise zwischen 0,5 und 1 Gew.-% vorliegt.

5. Zusammensetzung nach einem der vorangehenden Ansprüche, **dadurch gekennzeichnet, dass** die Verbindung, die den Haarausfall verringert oder das (Nach)Wachsen der Haare begünstigt, ausgewählt ist unter den Mitteln, die die Lebensfähigkeit des Haarbalgs verbessern.

6. Zusammensetzung nach einem der Ansprüche 1 bis 4, **dadurch gekennzeichnet , dass** die Verbindung, die den Haarausfall verringert oder das (Nach)Wachsen der Haare begünstigt, unter Vitaminen, gezuckerten Energiemitteln und Ginseng ausgewählt ist.

7. Zusammensetzung nach einem der Ansprüche 1 bis 4, **dadurch gekennzeichnet, dass** die Verbindung, die den Haarausfall verringert oder das (Nach)Wachsen der Haare begünstigt, unter Biotin, Aminexil, Minoxidil und dem Tripeptid KPV und seinen Salzen ausgewählt ist.

8. Zusammensetzung nach einem der Ansprüche 1 bis 4, **dadurch gekennzeichnet, dass** die Verbindung, die den Haarausfall verringert oder das (Nach)Wachsen der Haare begünstigt, unter den Verbindungen ausgewählt ist, die auf systemischem Weg auf den Metabolismus der Androgene wirken.

9. Zusammensetzung nach einem der Ansprüche 1 bis 4, **dadurch gekennzeichnet , dass** die Verbindung, die den Haarausfall verringert oder das (Nach)Wachsen der Haare begünstigt, vorzugsweise in einer Konzentration zwischen 0,01 Gew.-% und 10 Gew.-%, bezogen auf die ganze Zusammensetzung, vorzugsweise zwischen 0,1% und 5%, vorliegt.

10. Kosmetisches Behandlungsverfahren für Haare und/oder die behaarte Kopfhaut, **dadurch gekennzeichnet, dass** man eine Zusammensetzung nach einem der Ansprüche 1 bis 9 auf die Haare und/oder die behaarte Kopfhaut aufträgt.
